(19) Europäisches Patentamt
European Patent Office
Office européen des brevets

(11) EP 0 680 743 B1

(12) **FASCICULE DE BREVET EUROPEEN**

(45) Date de publication et mention de la délivrance du brevet:
**09.04.1997 Bulletin 1997/15**

(51) Int. Cl.[6]: **A61K 7/06**, A61K 7/032

(21) Numéro de dépôt: **95400667.2**

(22) Date de dépôt: **24.03.1995**

(54) **Composition pour le traitement et la protection des cheveux, cils et sourcils à base de céramides et de polymères de vinylpyrrolidone**

Zusammensetzung zur Behandlung und zum Schutz von Haaren, Wimpern und Augenbrauen auf der Basis von Ceramiden und Vinylpyrrolidonpolymeren

Composition for protecting and treating hair, eyelashes and eyebrows based on ceradides and vinylpyrrolidone polymers

(84) Etats contractants désignés:
**AT BE CH DE ES FR GB IT LI NL SE**

(30) Priorité: **02.05.1994 FR 9405314**

(43) Date de publication de la demande:
**08.11.1995 Bulletin 1995/45**

(73) Titulaire: **L'OREAL**
**75008 Paris (FR)**

(72) Inventeur: **Cretois, Isabelle**
**F-92110 Clichy (FR)**

(74) Mandataire: **Tezier Herman, Béatrice**
**L'OREAL,**
**Département Propriété Industrielle,**
**90, rue du Gal Roguet**
**92583 Clichy Cédex (FR)**

(56) Documents cités:
**WO-A-93/02656**
**WO-A-93/14024**
**WO-A-94/02115**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 680 743 B1

Printed by Rank Xerox (UK) Business Services
2.14.2/3.4

## Description

La présente invention concerne des compositions destinées au traitement et à la protection des phanères kératiniques, en particulier les cheveux et les cils contenant, dans un milieu cosmétiquement acceptable, au moins un céramide et/ou glycocéramide et au moins un polymère de vinylpyrrolidone.

L'invention concerne également l'utilisation de telles compositions pour le traitement non lavant des phanères kératiniques, en particulier les cheveux et les cils et le procédé de traitement cosmétique mettant en oeuvre de telles compositions.

On connaît déjà dans l'état de la technique, par exemple dans le document WO 93/14024, des formulations permettant de fixer et de conditionner les cheveux et/ou les cils. On a déjà utilisé dans ce but des polymères de vinylpyrrolidone qui ont pour avantage de maintenir les ondulations des cheveux et/ou des cils dans des conditions d'humidité élevée.

Selon la demande de brevet EP-A-0 278 505, on connaît également les céramides et les glycocéramides qui ont déjà été associés à des esters de cholestérol dans le but de protéger la fibre capillaire. Les céramides n'ont cependant jamais été décrits pour conférer une bonne tenue à la chevelure.

Or, la demanderesse vient maintenant de découvrir, après d'importantes recherches, que l'association de céramides et/ou de glycocéramides avec des polymères de vinylpyrrolidone, conduisait de façon inattendue et surprenante à des propriétés particulièrement intéressantes, notamment une amélioration remarquable de la tenue de la coiffure dans le temps.

Cette découverte est à la base de la présente invention.

La présenté invention a donc pour objet de nouvelles compositions cosmétiques non lavantes pour le traitement et la protection des phanères kératiniques, en particulier les cheveux et les cils, contenant dans un milieu cosmétiquement acceptable non détergent, au moins un céramide et/ou glycocéramide et au moins un polymère de vinylpyrrolidone, lesdites compositions contenant moins de 4% d'agents tensio-actifs anioniques et/ou amphotères et/ou zwittérioniques.

Un autre objet de l'invention concerne l'utilisation de ces compositions pour le traitement non lavant des phanères kératiniques, en particulier les cheveux et les cils. On convient ici de définir un traitement non lavant par un traitement qui met en oeuvre une composition renfermant moins de 4% en poids d'agents tensioactifs anioniques et/ou amphotères et/ou zwittérioniques.

Un autre objet de l'invention concerne également le procédé de traitement cosmétique mettant en oeuvre de telles compositions.

D'autres caractéristiques, aspects, objets et avantages de l'invention apparaîtront encore plus clairement à la lecture de la description et des exemples qui suivent. Par phanères kératiniques, on comprendra plus particulièrement les cheveux, les cils, les sourcils et les ongles.

Les céramides et/ou glycocéramides sont connus en eux-mêmes et sont des molécules naturelles ou synthétiques pouvant répondre à la formule générale :

$$R_3CHOH - CH - CH_2OR_2$$
$$|$$
$$NH$$
$$|$$
$$C{=}O \qquad (I)$$
$$|$$
$$R_1$$

dans laquelle :

- $R_1$ désigne un radical alkyle, linéaire ou ramifié, saturé ou insaturé, dérivé d'acides gras en $C_{14}$-$C_{30}$, ce radical pouvant être substitué par un groupement hydroxyle en position $\alpha$, ou un groupement hydroxyle en position $\omega$ estérifié par un acide gras saturé ou insaturé en $C_{16}$-$C_{30}$ ;
- $R_2$ désigne un atome d'hydrogène ou un radical (glycosyle)$_n$, (galactosyle)$_m$ ou sulfogalactosyle, dans lesquels n est un entier variant de 1 à 4 et m est un entier variant de 1 à 8 ;

- $R_3$ désigne un radical hydrocarboné en $C_{15}$-$C_{26}$, saturé ou insaturé en position $\alpha$, ce radical pouvant être substitué par un ou plusieurs radicaux alkyle en $C_1$-$C_{14}$ ; ou $R_3$ désigne un radical $\alpha$-hydroxyalkyle en $C_{15}$-$C_{26}$, le groupement hydroxyle étant éventuellement estérifié par un $\alpha$-hydroxyacide en $C_{16}$-$C_{30}$.

Les céramides préférées dans le cadre de la présente invention sont celles décrites par DOWNING dans Arch. Dermatol, Vol. 123, 1381-1384, 1987, ou celles décrites dans le brevet français FR-2 673 179, dont les structures peuvent être les suivantes :

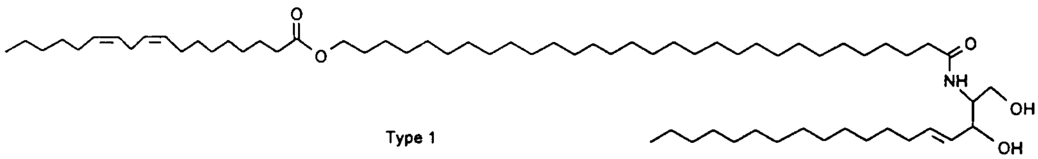
Type 1

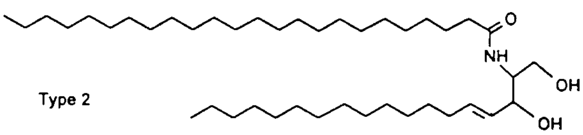
Type 2

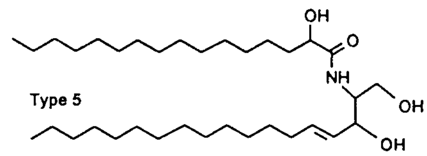
Type 5

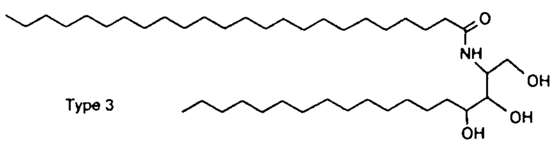
Type 3

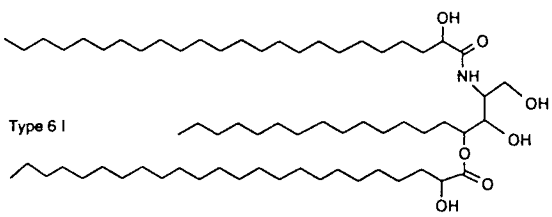
Type 6 I

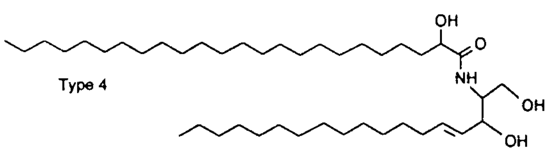
Type 4

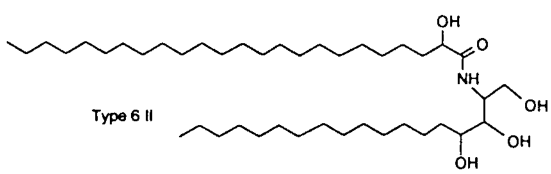
Type 6 II

Les céramides plus particulièrement préférés selon l'invention sont les composés de formule (I) pour lesquels $R_1$ désigne un alkyle saturé ou insaturé dérivé d'acides gras en $C_{16}$-$C_{22}$ ; $R_2$ désigne un atome d'hydrogène ; et $R_3$ désigne un radical linéaire saturé en $C_{15}$.

De tels composés sont par exemple :

- la N-linoléoyldihydrosphingosine,
- la N-oléoyldihydrosphingosine,
- la N-palmitoyldihydrosphingosine,
- la N-stéaroyldihydrosphingosine,
- la N-béhénoyldihydrosphingosine,

ou les mélanges de ces composés.

Encore plus préférentiellement, on utilise les composés de formule (I) pour lesquels $R_1$ désigne un radical alkyle saturé ou insaturé dérivé d'acides gras ; $R_2$ désigne un radical galactosyle ou sulfogalactosyle ; et $R_3$ désigne un grou-

pement $-CH=CH-(CH_2)_{12}-CH_3$.

A titre d'exemple, on peut citer le produit constitué d'un mélange de ces composés, vendu sous la dénomination commerciale GLYCOCER® par la société WAITAKI INTERNATIONAL BIOSCIENCES.

La concentration en ces céramides et/ou glycocéramides peut varier entre 0,005% et 5% en poids environ par rapport au poids total de la composition, et de préférence entre 0,01 et 3%environ.

Les polymères de vinylpyrrolidone (PVP) utilisables conformément à l'invention sont choisis plus particulièrement parmi les polymères suivants :

a) les polymères de vinylpyrrolidone comportant des motifs Méthacrylate de diméthylaminoéthyle ; on peut citer parmi ceux-ci :

- le copolymère vinylpyrrolidone / Méthacrylate de diméthylaminoéthyle (20/80 en poids) vendu sous la dénomination commerciale COPOLYMER 845® par la société I.S.P.
- les copolymères vinylpyrrolidone / Méthacrylate de diméthylaminoéthyle quaternisés par du sulfate de diéthyle, vendus sous les dénominations GAFQUAT 734®, 755®, 755 S® et 755 L® par la société I.S.P.
- les PVP / Méthacrylate de diméthylaminoéthyle / Polyuréthane hydrophile, vendus sous la dénomination commerciale PECOGEL GC-310® par la société U.C.I.B. ou encore sous les dénominations AQUAMERE C 1031® et C 1511® par la société BLAGDEN CHEMICALS,
- les PVP / Méthacrylate de diméthylaminoéthyle / Oléfine en C8 à C16, quaternisés ou non quarternisés, vendus sous les dénominations GANEX ACP 1050 à 1057, 1062 à 1069, 1079 à 1086, par la société I.S.P.
- le PVP / Méthacrylate de diméthylaminoéthyle / Vinylcaprolactame, vendu sous la dénomination GAFFIX VC 713® par la société I.S.P.

b) les polymères de vinylpyrrolidone comportant des motifs Méthacrylamidopropyltriméthylammonium ( M.A.P.T.A.C. ), parmi lesquels on peut citer notamment :

- les copolymères vinylpyrrolidone / M.A.P.T.A.C., vendus sous les dénominations commerciales GAFQUAT ACP 1011® et GAFQUAT HS 100® par la société I.S.P.
- les terpolymères vinylpyrrolidone / M.A.P.T.A.C. / Vinylcaprolactame, vendus sous les dénominations POLYMER ACP 1059®, 1060®, et 1156® par la société I.S.P.

c) les polymères de vinylpyrrolidone comportant des motifs Méthylvinylimidazolium, et parmi lesquels on peut citer plus particulièrement :

- les PVP / Chlorure de méthylvinylimidazolium, vendus sous les dénominations LUVIQUAT FC 370®, FC 550®, FC 905®, HM 552® par la société B.A.S.F.
- le PVP / Chlorure de méthylvinylimidazolium / Vinylimidazole, vendu sous la dénomination LUVIQUAT 8155® par la société B.A.S.F.
- le PVP / Méthosulfate de méthylvinylimidazolium, vendu sous la dénomination LUVIQUAT MS 370® par la société B.A.S.F.

La concentration en ces polymères de vinylpyrrolidone peut varier entre 0,05 et 5% en poids environ par rapport au poids total de la composition, et de préférence entre 0,1 et 3% environ.

Le milieu cosmétiquement acceptable est de préférence constitué par de l'eau ou un mélange d'eau et de solvants cosmétiquement acceptables tels que des monoalcools, des polyalcools, des éthers de glycol ou des esters d'acides gras, qui peuvent être utilisés seuls ou en mélange.

On peut citer plus particulièrement les alcools inférieurs tels que l'éthanol, l'isopropanol, les polyalcools tels que le diéthylèneglycol, les éthers de glycol, les alkyléthers de glycol ou de diéthylèneglycol.

Le pH des compositions selon l'invention est généralement compris entre 2 et 9, et en particulier entre 3 et 8. Il peut être ajusté à la valeur choisie au moyen d'agents alcalinisants ou acidifiants habituellement utilisés en cosmétique pour ce type d'application.

Ces compositions peuvent en outre contenir des agents tensio-actifs de type nonionique et/ou cationique, bien connus dans l'état de la technique, et ceci dans des proportions généralement comprises entre environ 0,1 et 10% en poids par rapport à l'ensemble de la composition.

Les compositions selon l'invention peuvent encore contenir en plus des céramides et/ou glycocéramides et des polymères de vinylpyrrolidone définis ci-dessus, des agents épaississants, des agents conservateurs, des séquestrants, des adoucissants, des parfums, des colorants, des agents modificateurs de viscosité, des agents modificateurs de mousse, des agents moussants, des stabilisateurs de mousse, des agents nacrants, des agents hydratants, des agents antipelliculaires, des agents antiséborrhéiques, des filtres solaires, des protéines, des vitamines, des plasti-

fiants, des $\alpha$-hydroxyacides, des électrolytes, des agents propulseurs et des parfums.

Les compositions selon l'invention peuvent également contenir d'autres agents conditionneurs. Ceux-ci peuvent alors être choisis parmi les huiles et les cires naturelles ou synthétiques, les alcools gras, les esters d'alcools polyhydriques, les glycérides, les gommes et résines de silicone ou les mélanges de ces différents composés.

Les compositions utilisées selon l'invention sont par exemple des composition capillaires rincées ou non rincées, des compositions de maquillage des cils ou des sourcils telles que les mascaras.

Les compositions selon l'invention peuvent être appliquées avant ou après un shampooing, une coloration, une décoloration, une permanente ou être appliquées entre les étapes de la réduction et de la fixation d'une permanente ou d'un défrisage.

Les compositions appliquées sur les phanères kératiniques peuvent se présenter sous des formes diverses, telles que sous forme d'émulsions, de dispersions, de solutions, de fluides plus ou moins épaissis, de gels, de crèmes et de gels-crèmes.

Elles peuvent être utilisées en l'état ou être diluées avant utilisation.

En particulier, elles peuvent être conditionnées sous pression en flacon aérosol en présence d'un agent propulseur et former une mousse ou en flacon pompe pour être délivrées sous forme d'un spray.

Le procédé non lavant de traitement cosmétique et de protection des phanères kératiniques est caractérisé par le fait que l'on applique sur des phanères kératiniques, en particulier les cheveux et les cils une composition contenant au moins un céramide et/ ou un glycocéramide et au moins un un polymère de vinylpyrrolidone, et qu'après un éventuel temps de pose, on procède éventuellement à un rinçage.

Des exemples concrets illustrant l'invention vont maintenant être donnés.

**EXEMPLE 1**

On a préparé une mousse de coiffage de composition suivante :

| | |
|---|---|
| - Hydroxyéthylcellulose modifiée par une chaîne cétyle, vendue sous la dénomination NATROSOL PLUS GRADE 330 CS® par la société AQUALON | 0,1g |
| - Copolymère de vinylpyrrolidone et de Méthacrylate de diméthylaminoéthyle, quaternisé par du sulfate de diéthyle, vendu en solution à 50% dans l'éthanol, par la société I.S.F. sous la dénomination GAFQUAT 734® | 0,75g MA |
| - Butanediol oxypropyléné à 10 moles d'oxyde de propylène vendu sous le nom de MACOL 57® par la société MAZER | 0,5g |
| - Phosphate de cétyl diméthyl (2'hydroxyéthyl) ammonium en solution aqueuse à 30% | 0,15g MA |
| -Octylphénol à 10 moles d'oxyde d'éthylène vendu sous le nom de IGEPAL O® par la société RHONE POULENC | 0,3g |
| - Alcool polyvinylique vendu sous la dénomination de COVOL 9740® par la société C.P.C. Int. | 0,6g |
| - N-oleoyl dihydrosphingosine | 0,1g |
| - Méthosulfate de 1-méthyl 2-suif 3-suifamidoéthyl imidazolinium à 75% dans le propylène glycol | 0,375g |
| - Conservateur | qs |
| -Parfum | qs |
| - eau déminéralisée qsp | 100g |

<u>Conditionnement en aérosol :</u>

90g de la composition ci-dessus ont été conditionnés dans un récipient aéosol en présence de 10g d'un mélange ternaire de n-butane, isobutane et propane, (23/55/22), vendu sous la dénomination "AEROGAZ 3,2 N® par la société ELF AQUITAINE.

Cette mousse a été appliquée sur des cheveux mouillés et a conféré, après séchage de ces derniers, une bonne tenue dans le temps à la coiffure.

**EXEMPLE 2**

On a préparé une mousse de coiffage de composition suivante :

| | |
|---|---|
| - Nonoxynyl hydroxyéthylcellulose, vendue sous la dénomination AMERCELL POLYMER HM 1500® par la société AMERCHOL | 0,1g |
| - Copolymère de vinylpyrrolidone et de Chlorure de méthylvinylimidazolium, (70/30), vendu en solution aqueuse à 40% par la société B.A.S.F., sous la dénomination LUVIQUAT FC 370® | 0,2g MA |
| - Huile de ricin hydrogénée, oxyéthylénée par 40 moles d'oxyde d'éthylène, vendue sous la dénomination CREMOPHOR RH 40® par la société B.A.S.F | 0,25g |
| - Butanediol oxypropyléné à 10 moles d'oxyde de propylène vendu sous le nom de MACOL 57® par la société MAZER | 0,4g |
| - Octylphénol à 10 moles d'oxyde d'éthylène vendu sous le nom de IGEPAL O® par la société RHONE POULENC | 0,4g |
| - Alcool polyvinylique vendu sous la dénomination de COVOL 9740® par la société C.P.C. Int. | 0,8g |
| - N-oleoyl dihydrosphingosine | 0,05g |
| - Conservateur | qs |
| -Parfum | qs |
| - eau déminéralisée qsp | 100g |

Conditionnement en aérosol :

90g de la composition ci-dessus sont conditionnés dans un récipient aéosol en présence de 10g d'un mélange ternaire de n-butane, isobutane et propane, (23/55/22), vendu sous la dénomination de "AEROGAZ 3,2 N® par la société ELF AQUITAINE

Cette mousse a été appliquée sur les cheveux mouillés et a conféré, après séchage de ces derniers, une bonne tenue dans le temps à la coiffure.

**EXEMPLE 3**

On a préparé une lotion (A) de mise en plis selon l'invention de composition suivante :

| | |
|---|---|
| - Copolymère de vinylpyrrolidone et de Chlorure de méthylvinylimidazolium, (70/30), vendu en solution aqueuse à 40% par la société B.A.S.F., sous la dénomination LUVIQUAT FC 370® | 0,5g MA |
| - N-oleoyl dihydrosphingosine | 0,05g |
| - Méthosulfate de 1-méthyl 2-suif 3-suifamidoéthyl imidazolinium à 75% dans le propylène glycol (REWOQUAT W 75 PG® de REWO) | 0,5g MA |
| - Eau déminéralisée qsp | 100g |

On a préparé une lotion (B) de mise en plis non conforme à l'invention (lotion (A) sans céramide) de composition suivante :

| | |
|---|---|
| - Copolymère de vinylpyrrolidone et de Chlorure de méthylvinylimidazolium, (70/30), vendu en solution aqueuse à 40% par la société B.A.S.F., sous la dénomination LUVIQUAT FC 370® | 0,5g MA |
| - Méthosulfate de 1-méthyl 2-suif 3-suifamidoéthyl imidazolinium à 75% dans le propylène glycol (REWOQUAT W 75 PG® de REWO) | 0,5g MA |
| - Eau déminéralisée qsp | 100g |

Le mode opératoire est le suivant : on applique sur des mèches de cheveux soit la lotion (A), soit la lotion (B); puis on enroule les mèches ainsi traitées sur des bigoudis; puis on fait sécher la mèche, enfin on déroule la mèche du bigoudis.

Pour quantifer l'efficacité de la composition selon l'invention, on a d'abord mesuré la longueur initiale $L_0$ (en cm) de la mèche de cheveux avant le traitement à la vapeur (longueur mesurée entre les racines et les pointes sur mèche suspendue verticalement); de la même manière, on a mesuré ensuite la longueur $L_1$ de cette même mèche juste en fin de traitement; et enfin, on a mesuré la longueur $L_2$ de cette mèche 5 heures après le traitement.

On définit la tenue (en %) de la mise en plis par le rapport

$$r=\frac{L_0-L_2}{L_0-L_1} \times 100$$

La mise en plis sera d'autant meilleure que ce rapport sera élevé.

Les résultats obtenus ont été les suivants :

Pour la lotion (A) selon l'invention, la tenue de la mise en plis est de 48,3% alors que pour la lotion (B), il est de 43,5% : soit une augmentation de plus de 11% avec la lotion (A).

**EXEMPLE 4**

On a préparé un mascara conforme à l'invention de composition suivante :

| | |
|---|---|
| Phase A : | |
| - Cire d'abeille | 6,9 g |
| - Cire de carnauba | 4,16 g |
| - Paraffine | 11,4 g |
| - Acide stéarique | 7,7 g |
| - N-oléoyl dihydrosphingosine | 0,2 g |
| Phase B : | |
| - Oxyde de fer noir | 5,55 g |
| Phase C: | |
| - Triéthanolamine | 3,8 g |
| - Gomme arabique | 4,5 g |
| - Polyvinylpyrrolidone (POVIDONE® de ISP) | 0,5 g |
| - Conservateurs qs | |
| - Eau déminéralisée qs | 100 g |

On fait fondre la phase A à 80°C puis on introduit la phase B que l'on disperse à l'aide d'un homogénéïsateur pendant 30 minutes.

On prépare la phase C en introduisant les trois premiers composants de cette phase dans l'eau portée à 75°C. On réalise ensuite une émulsion en mélangeant la phase C à la phase A+B.

On a appliqué la composition de mascara sur les cils. Les cils présentent une bonne tenue.

## Revendications

1. Composition destinée au traitement et à la protection des phanères kératiniques, caractérisée par le fait qu'elle contient, dans un milieu cosmétiquement acceptable, au moins un céramide et/ou glycocéramide et au moins un polymère de vinylpyrrolidone, ladite composition contenant moins de 4% en poids d'agents tensio-actifs anioniques et/ou amphotères et/ou zwittérioniques.

2. Composition selon la revendication 1, caractérisée par le fait que les céramides et/ou glycocéramides sont choisis parmi les composés de formule générale (I) suivante :

$$R_3CHOH\text{--}CH\text{--}CH_2OR_2$$

$$\quad\quad\quad\quad\quad |$$

$$\quad\quad\quad\quad\quad NH$$

$$\quad\quad\quad\quad\quad | \qquad\qquad\qquad\qquad (I)$$

$$\quad\quad\quad\quad\quad C{=}O$$

$$\quad\quad\quad\quad\quad |$$

$$\quad\quad\quad\quad\quad R_1$$

dans laquelle :

- $R_1$ désigne un radical alkyle, linéaire ou ramifié, saturé ou insaturé, dérivé d'acides gras en $C_{14}$-$C_{30}$, ce radical pouvant être substitué par un groupement hydroxyle en position $\alpha$, ou un groupement hydroxyle en position $\omega$ estérifié par un acide gras saturé ou insaturé en $C_{16}$-$C_{30}$ ;
- $R_2$ désigne un atome d'hydrogène ou un radical (glycosyle)$_n$, (galactosyle)$_m$ ou sulfogalactosyle, dans lesquels n est un entier variant de 1 à 4 et m est un entier variant de 1 à 8 ;
- $R_3$ désigne un radical hydrocarboné en $C1_5$-$C_{26}$, saturé ou insaturé en position $\alpha$, ce radical pouvant être substitué par un ou plusieurs radicaux alkyle en $C_1$-$C_{14}$ ; ou $R_3$ désigne un radical $\alpha$-hydroxyalkyle en $C_{15}$-$C_{26}$, le groupement hydroxyle étant éventuellement estérifié par un $\alpha$-hydroxyacide en $C_{16}$-$C_{30}$.

3. Composition selon la revendication 1 ou 2, caractérisée par le fait que les polymères de vinylpyrrolidone sont choisis parmi :

   a) les polymères de vinylpyrrolidone comportant des motifs Méthacrylate de diméthylaminoéthyle, quaternisés ou non,
   b) les polymères de vinylpyrrolidone comportant des motifs Méthacrylamidopropyltriméthylammonium,
   c) les polymères de vinylpyrrolidone comportant des motifs Méthylvinylimidazolium.

4. Composition selon l'une quelconque des revendications 1 à 3, caractérisée par le fait que les céramides et/ou glycocéramides sont présents dans des proportions comprises entre 0,005% et 5% en poids environ par rapport au poids total de la composition, et de préférence entre 0,01 et 3% environ.

5. Composition selon l'une quelconque des revendications 1 à 4, caractérisée par le fait que les polymères de vinylpyrrolidone sont présents dans des proportions comprises entre 0,05 et 5% en poids environ par rapport au poids total de la composition, et de préférence entre 0,1 et 3% environ.

6. Composition selon l'une quelconque des revendications 1 à 5, caractérisée par le fait que le milieu cosmétiquement acceptable est constitué par de l'eau, un mélange d'eau et de solvants cosmétiquement acceptables choisis parmi les monoalcools, les polyalcools, les éthers de glycol ou les esters d'acides gras, utilisés seuls ou en mélange.

**7.** Composition selon l'une quelconque des revendications 1 à 6, caractérisée par le fait qu'elle contient également des agents tensio-actifs de type non-ionique et/ou cationique, des agents épaississants, des agents conservateurs, des séquestrants, des adoucissants, des parfums, des colorants, des agents modificateurs de viscosité, des agents modificateurs de mousse, des agents moussants, des stabilisateurs de mousse, des agents nacrants, des agents hydratants, des agents antipelliculaires, des agents antiséborrhéiques, des filtres solaires, des protéines, des vitamines, des plastifiants, des $\alpha$-hydroxyacides, des électrolytes, des agents propulseurs, des parfums, et d'autres agents conditionneurs.

**8.** Composition selon l'une quelconque des revendications 1 à 7, caractérisée par le fait qu'elle se présente sous forme de fluide plus ou moins épaissi, de gel, de crème, de gel-crème, de spray ou de mousse.

**9.** Composition selon l'une quelconque des revendications 1 à 8, caractérisée par le fait que les phanères kératiniques sont des cheveux, des cils ou des sourcils.

**10.** Composition selon la revendication 9, caractérisée par le fait que les phanères kératiniques sont des cheveux.

**11.** Procédé non lavant de traitement cosmétique et de protection des phanères kératiniques, caractérisé par le fait que l'on applique sur des phanères kératiniques une composition telle que définie à l'une quelconque des revendications 1 à 10, et qu'après un éventuel temps de pose, on procède éventuellement à un rinçage.

**12.** Utilisation d'une composition telle que définie à l'une quelconque des revendications 1 à 10, pour conduire un traitement non lavant des phanères kératiniques.

**13.** Utilisation selon la revendication 12, caractérisée en ce que ledit traitement est destiné à améliorer la tenue des phanères kératiniques dans le temps.

**Claims**

**1.** Composition intended for the treatment and the protection of the keratinous exoskeletal parts, characterized in that it contains, in a cosmetically acceptable medium, at least one ceramide and/or glycoceramide and at least one vinylpyrrolidone polymer, the said composition containing less than 4 % by weight of anionic and/or amphoteric and/or zwitterionic surface-active agents.

**2.** Composition according to Claim 1, characterized in that the ceramides and/or glycoceramides are chosen from the compounds of the following general formula (I):

$R_3CHOH{-}CH{-}CH_2OR_2$
I
NH
I
C=O
I
$R_1$

(I)

in which:

- $R_1$ denotes a saturated or unsaturated, linear or branched alkyl radical derived from $C_{14}$-$C_{30}$ fatty acids, it being possible for this radical to be substituted by a hydroxyl group in $\alpha$ position or a hydroxyl group in $\omega$ position esterified by a $C_{16}$-$C_{30}$ saturated or unsaturated fatty acid;
- $R_2$ denotes a hydrogen atom or a $(glycosyl)_n$, $(galactosyl)_m$ or sulphogalactosyl radical, in which n is an integer varying from 1 to 4 and m is an integer varying from 1 to 8;
- $R_3$ denotes a saturated or $\alpha$-unsaturated $C_{15}$-$C_{26}$ hydrocarbon radical, it being possible for this radical to be substituted by one or more $C_1$-$C_{14}$ alkyl radicals; or $R_3$ denotes a $C_{15}$-$C_{26}$ $\alpha$-hydroxyalkyl radical, the hydroxyl group being optionally esterified by a $C_{16}$-$C_{30}$ $\alpha$-hydroxy acid.

**3.** Composition according to Claim 1 or 2, characterized in that the vinylpyrrolidone polymers are chosen from:

a) vinylpyrrolidone polymers containing dimethylaminoethyl methacrylate units, quaternized or otherwise,
b) vinylpyrrolidone polymers containing methacrylamidopropyltrimethylammonium units,
c) vinylpyrrolidone polymers containing methylvinylimidazolium units.

**4.** Composition according to any one of Claims 1 to 3, characterized in that the ceramides and/or glycoceramides are present in proportions of approximately between 0.005 % and 5 % by weight relative to the total weight of the composition, and preferably approximately between 0.01 and 3 %.

**5.** Composition according to any one of Claims 1 to 4, characterized in that the vinylpyrrolidone polymers are present in proportions of approximately between 0.05 and 5 % by weight relative to the total weight of the composition, and preferably approximately between 0.1 and 3 %.

**6.** Composition according to any one of Claims 1 to 5, characterized in that the cosmetically acceptable medium consists of water, a mixture of water and of cosmetically acceptable solvents chosen from monoalcohols, polyalcohols, glycol ethers or fatty acid esters, employed by themselves or mixed.

**7.** Composition according to any one of Claims 1 to 6, characterized in that it also contains surface-active agents of nonionic and/or cationic type, thickening agents, preserving agents, seguestrants, softeners, perfumes, colorants, viscosity modifiers, foam modifiers, foaming agents, foam stabilizers, pearlescent agents, hydrating agents, antidandruff agents, antiseborrhoeic agents, sunscreens, proteins, vitamins, plasticizers, $\alpha$-hydroxyacids, electrolytes, propellants, perfumes and other conditioning agents.

**8.** Composition according to any one of Claims 1 to 7, characterized in that it is in the form of a more or less thickened fluid, gel, cream, gel-cream, spray or mousse.

**9.** Composition according to any one of Claims 1 to 8, characterized in that the keratinous exoskeletal parts are hair, eyelashes or eyebrows.

**10.** Composition according to Claim 9, characterized in that the keratinous exoskeletal parts are hair.

**11.** Nonwashing process for the cosmetic treatment and protection of the keratinous exoskeletal parts, characterized in that a composition as defined in any one of Claims 1 to 10 is applied to the keratinous exoskeletal parts and that, after an optional exposure time, rinsing is optionally carried out.

**12.** Use of a composition as defined in any one of Claims 1 to 10 for conducting a nonwashing treatment of the keratinous exoskeletal parts.

**13.** Use according to Claim 12, characterized in that the said treatment is intended to improve the shape-retention of the keratinous exoskeletal parts over time.

**Patentansprüche**

**1.** Zusammensetzung, die zur Behandlung und zum Schutz von Keratinsubstanzen bestimmt ist,
**dadurch gekennzeichnet, daß**
sie in einem kosmetisch akzeptablen Medium mindestens ein Ceramid und/oder Glykoceramid und mindestens ein Vinylpyrrolidonpolymer enthält, wobei die Zusammensetzung weniger als 4 Gew.-% anionische und/oder amphotere und/oder zwitterionische grenzflächenaktive Stoffe enthält.

**2.** Zusammensetzung nach Anspruch 1, dadurch gekennzeichnet, daß die Ceramide und/oder Glykoceramide unter den Verbindungen der folgenden allgemeinen Formel (I):

$R_3CHOH - CH - CH_2OR_2$
I
NH
I
C=O
I
$R_1$

(I)

ausgewählt sind, worin bedeuten:

- $R_1$ eine lineare oder verzweigte, gesättigte oder ungesättigte, von $C_{14-30}$-Fettsäuren abgeleitete Alkylgruppe, wobei die Gruppe mit einer Hydroxygruppe in $\alpha$-Stellung oder einer Hydroxygruppe in $\omega$-Stellung, die mit einer gesättigten oder ungesättigten $C_{16-30}$-Fettsäure verestert ist, substituiert sein kann;

- $R_2$ ein Wasserstoffatom oder eine Gruppe $(Glykosyl)_n$, $(Galactosyl)_m$ oder Sulfogalactosyl, wobei n eine ganze Zahl im Bereich von 1 bis 4 und m eine ganze Zahl im Bereich von 1 bis 8 ist;

- $R_3$ eine gesättigte oder in $\alpha$-Stellung ungesättigte Kohlenwasserstoffgruppe mit 15 bis 26 Kohlenstoffatomen, wobei die Gruppe mit einer oder mehreren $C_{1-14}$-Alkylgruppen substituiert sein kann; oder eine $\alpha$-Hydroxyalkylgruppe mit 15 bis 26 Kohlenstoffatomen, wobei die Hydroxygruppe gegebenenfalls mit einer $\alpha$-Hydroxysäure mit 16 bis 30 Kohlenstoffatomen verestert sein kann.

**3.** Zusammensetzung nach Anspruch 1 oder 2, dadurch gekennzeichnet, daß die Vinylpyrrolidonpolymere ausgewählt sind unter:

a) Vinylpyrrolidonpolymeren, die Dimethylaminoethylmethacrylat-Einheiten enthalten, die gegebenenfalls quaternisiert sind.

b) Vinylpyrrolidonpolymere, die Methacrylamidopropyltrimethylammonium-Einheiten enthalten.

c) Vinylpyrrolidonpolymere, die Methylvinylimidazolium-Einheiten enthalten.

**4.** Zusammensetzung nach einem der Ansprüche 1 bis 3, dadurch gekennzeichnet, daß die Ceramide und/oder Glykoceramide in Anteilen im Bereich von etwa 0,005 bis 5 Gew.-%, bezogen auf das Gesamtgewicht der Zusammensetzung, und vorzugsweise von etwa 0,01 bis 3 Gew.-% vorliegen.

**5.** Zusammensetzung nach einem der Ansprüche 1 bis 4, dadurch gekennzeichnet, daß die Vinylpyrrolidonpolymere in Anteilen im Bereich von etwa 0,05 bis 5 Gew.-%, bezogen auf das Gesamtgewicht der Zusammensetzung, und vorzugsweise von etwa 0,1 bis 3 Gew.-% vorliegen.

**6.** Zusammensetzung nach einem der Ansprüche 1 bis 5, dadurch gekennzeichnet, daß das kosmetisch akzeptable Medium aus Wasser oder einem Gemisch aus Wasser und kosmetisch akzeptablen Lösungsmitteln besteht, welche unter den Monoalkoholen, Polyalkoholen, Glykolethern oder Fettsäureestern ausgewählt sind, die allein oder im Gemisch verwendet werden.

**7.** Zusammensetzung nach einem der Ansprüche 1 bis 6, dadurch gekennzeichnet, daß sie ferner nichtionische und/oder kationische grenzflächenaktive Stoffe, Verdickungsmittel, Konservierungsmittel, Maskierungsmittel, reizlindernde Mittel, Parfums, Färbemittel, Mittel, die die Viskosität modifizieren, Mittel, die die Schaumfähigkeit modifizieren, Schaumbildner, Schaumstabilisatoren, Perlglanzmittel, Hydratisierungsmittel, Mittel gegen Schuppen, Mittel gegen Seborrhoe, Sonnenschutzfilter, Proteine, Vitamine, Plastifizierungsmittel, $\alpha$-Hydroxysäuren, Elektrolyte, Treibmittel, Parfums und weitere Konditionierungsmittel enthalten.

8. Zusammensetzung nach einem der Ansprüche 1 bis 7, dadurch gekennzeichnet, daß sie als mehr oder weniger dickflüssiges Fluid, als Gel, Creme, Gel-Creme, Spray oder Schaum vorliegt.

9. Zusammensetzung nach einem der Ansprüche 1 bis 8, dadurch gekennzeichnet, daß die Keratinsubstanzen Haare, Wimpern oder Augenbrauen sind.

10. Zusammensetzung nach Anspruch 9, dadurch gekennzeichnet, daß die Keratinsubstanzen Haare sind.

11. Nicht reinigendes Verfahren zur kosmetischen Behandlung und zum Schutz von Keratinsubstanzen, dadurch gekennzeichnet, daß auf die Keratinsubstanzen eine Zusammensetzung nach einem der Ansprüche 1 bis 10 aufgetragen wird und nach einer gegebenenfalls vorhandenen Einwirkzeit gegebenenfalls gespült wird.

12. Verwendung einer Zusammensetzung nach einem der Ansprüche 1 bis 10 zur Durchführung einer nicht reinigenden Behandlung von Keratinsubstanzen.

13. Verwendung nach Anspruch 12, dadurch gekennzeichnet, daß die Behandlung dazu bestimmt ist, das Verhalten der Keratinsubstanzen über der Zeit zu verbessern.